# EUROPÄISCHE PATENTSCHRIFT

(11) **EP 1 140 782 B1**
(45) Veröffentlichungstag und Bekanntmachung des Hinweises auf die Patenterteilung: **03.09.2003**
(21) Anmeldenummer: 99963455.3
(22) Anmeldetag: 09.12.1999
(51) Int. Cl.: C07C 68/08, C07C 69/96

(54) **VERFAHREN ZUR AUFARBEITUNG VON DIARYLCARBONATHALTIGEN REAKTIONSMISCHUNGEN**
METHOD FOR REPROCESSING REACTION MIXTURES CONTAINING DIARYL CARBONATE
PROCEDE PERMETTANT DE RETRAITER DES MELANGES DE REACTION RENFERMANT DES DIARYLCARBONATES

(30) Priorität: 22.12.1998 DE 19859295
(43) Veröffentlichungstag der Anmeldung: 10.10.2001
(73) Patentinhaber: Bayer Aktiengesellschaft, 51368 Leverkusen (DE)
(72) Erfinder: HESSE, Carsten, D-47918 Tönisvorst (DE); JANSEN, Ursula, D-47799 Krefeld (DE); RECHNER, Johann, D-47906 Kempen (DE); REISINGER, Claus-Peter, D-47798 Krefeld (DE); EEK, Rob, D-51061 Köln (DE); HALLENBERGER, Kaspar, D-51375 Leverkusen (DE); FRIEDRICH, Martin, D-51069 Köln (DE)
(86) Internationale Anmeldenummer: EP9909697
(87) Internationale Veröffentlichungsnummer: WO00037419

(56) Entgegenhaltungen:
- EP-A- 0 507 546
- EP-A- 0 749 955

## Beschreibung

Die vorliegende Erfindung betrifft ein Verfahren zur Aufarbeitung von Diarylcarbonat, aromatische Hydroxyverbindung, Wasser, Base, quaternäres Salz und gegebenenfalls weitere Katalysatorbestandteile enthaltenden Reaktionsmischungen, die bei der Herstellung von Diarylcarbonaten durch Direktcarbonylierung von aromatischen Hydroxyverbindungen erhalten werden. Bei dem erfindungsgemäßen Verfahren wird die Reaktionsmischung so schonend aufgearbeitet, daß das Katalysatorsystem kaum geschädigt wird und anschließend wieder in den Reaktionsschritt zurückgeführt werden kann.

EP-A 507 546 offenbart ein Verfahren zur Aufarbeitung diarylcarbonathaltiger Reaktionsmischungen, die durch Direktcarbonylierung aromatischer Hydroxy-verbindungen erhalten werden. Dabei werden aus dem Reaktionsgemisch zunächst die aromatische Hydroxyverbindung und in einem weiteren Schritt das gebildete Diarylcarbonat destillativ vollständig entfernt. Bei den hierfür notwendigen hohen Temperaturen und langen Verweilzeiten werden die in der Reaktionsmischung enthaltenen katalytisch wirksamen Komponenten vollständig desaktiviert bzw. zerstört. Weiterhin kommt es zu Ausbeuteverlusten durch Nebenreaktionen des Diarylcarbonats im Destillationssumpf.

Dokument EP-A-0 801 051 offenbart ein verfahren zur Herstellung von Diarylcarbonaten durch oxidative Carbonylierung der zugrundeliegenden aromatischen Hydroxyverbindungen in Gegenwart eines Platingruppenmetall enthaltenden Katalysators, eines Cokatalysators, eines quaternären Salzes und einer Base, wobei der Katalysator als stationär angeordneter oder in fluider Phase vorliegender Trägerkatalysator eingesetzt und die Reaktion in kondensierter Phase durchgeführt wird.

Es wurde nun ein Verfahren gefunden, bei dem die Reaktionsmischung so schonend aufgearbeitet wird, daß das Katalysatorsystem kaum geschädigt wird und anschließend wieder in den Reaktionsschritt zurückgeführt werden kann. Auch die Abtrennung des Diarylcarbonats erfolgt so schonend, daß kaum Nebenreaktionen auftreten.

Gegenstand der Erfindung ist ein Verfahren zur Aufarbeitung von Reaktionsmischungen aus der Herstellung von Diarylcarbonaten durch Direktcarbonylierung von aromatischen Hydroxyverbindungen, bei dem eine Diarylcarbonat, aromatische

Hydroxyverbindung, Wasser, Base, quaternäres Salz und gegebenenfalls weitere Katalysatorbestandteile enthaltende Reaktionsmischung in einer nur eine theoretische Trennstufe aufweisenden Destillationsapparatur bei Drücken von 1 bis 100 mbar und Temperaturen von 80 bis 160°C aufgetrennt wird in eine Diarylcarbonat, aromatische Hydroxyverbindung, Base, quaternäres Salz und gegebenenfalls weitere Katalysatorbestandteile enthaltende Flüssigphase und eine Diarylcarbonat, aromatische Hydroxyverbindung und Wasser enthaltende Gasphase, die Flüssigphase dem Reaktionsschritt der Direktcarbonylierung ohne weitere Aufarbeitung wieder zugeführt und die Gasphase anschließend weiter aufgearbeitet wird.

In einer weiteren Ausführungsform enthält die Reaktionsmischung außerdem noch einen Platinmetallkatalysator und einen Cokatalysator. Diese verbleiben nach der Auftrennung in der Flüssigphase und werden in dieser, gegebenenfalls nach Abtrennung von desaktivierten Katalysatorbestandteilen, dem Reaktionsschritt der Direktcarbonylierung ohne weitere Aufarbeitung wieder zugeführt.

Die Herstellung von Diarylcarbonaten durch Direktcarbonylierung von aromatischen Hydroxyverbindungen ist bekannt (siehe z.B. US-A 4,349,485, US-A 5,231,210, EP-A 667 336, EP-A 858 991, US-A 5,760,272).

Dabei wird eine aromatischen Hydroxyverbindung der Formel

R-O-H (I),

worin
- R: substituiertes oder nicht substituiertes C₆-C₁₂-Aryl, bevorzugt substituiertes oder nicht substituiertes Phenyl, besonders bevorzugt nicht substituiertes Phenyl bedeutet,
mit Kohlenmonoxid und Sauerstoff in Gegenwart eines Platinmetall-Katalysators, eines Cokatalysators, eines quaternären Salzes und einer Base bei einer Temperatur von 30 bis 200°C, bevorzugt 30 bis 150°C, besonders bevorzugt 40 bis 120°C und einem Druck von 1 bis 200 bar, bevorzugt 2 bis 100 bar, besonders bevorzugt 5 bis 50 bar umgesetzt.

Die Zusammensetzung der Reaktionsgase Kohlenmonoxid und Sauerstoff kann in weiten Konzentrationsgrenzen variiert werden, es wird jedoch zweckmäßigerweise ein CO:O₂-Molverhältnis (normiert auf CO) von 1:(0,001-1,0) bevorzugt 1:(0,01- 0,5) und besonders bevorzugt von 1:(0,02-0,3) eingestellt. Der Sauerstoffpartialdruck ist bei diesen Molverhältnissen groß genug, um hohe Raum-Zeit-Ausbeuten erreichen zu können und gleichzeitig keine explosionsfähigen Kohlenmonoxid/Sauerstoff-Gasgemische bilden zu können. Die Reaktionsgase unterliegen keinen besonderen Reinheitsanforderungen. So kann Synthesegas als CO-Quelle und Luft als O₂-Träger dienen, es ist jedoch darauf zu achten, daß keine Katalysatorgifte wie z.B.: Schwefel oder dessen Verbindungen eingetragen werden. Bevorzugt werden reines CO und reiner Sauerstoff verwendet.

Bei den umsetzbaren aromatischen Hydroxyverbindungen handelt es sich beispielsweise um Phenol, p-tert.-Butylphenol, o-, m- oder p-Kresol, o-, m- oder p-Chlorphenol, o-, m- oder p-Ethylphenol. o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol und Bisphenol A, bevorzugt um Phenol. Allgemein handelt es sich im Falle einer Substitution der aromatischen Hydroxyverbindung um 1 oder 2 Substituenten in der Bedeutung von C₁-C₄-Alkyl, C₁-C₄-Alkoxy, Fluor, Chlor oder Brom.

Einsetzbare Basen sind Alkali-, quatemäre Ammonium-, oder quaternäre Phosphoniumsalze von aromatischen Hydroxyverbindungen der Formel (I), wie beispielsweise Kaliumphenolat, Natriumphenolat, Tetrabutylammoniumphenolat. Alternativ können auch Trialkylamine wie Tributylamin, Diisopropylethylamin, DBU, DBN, oder auch andere Basen, z.B. Kalium-tert.-butanolat, Alkalimetallhydroxyde und Erdalkalimetallhydroxyde, verwendet werden.

Die Base wird in einer von der Stöchiometrie unabhängigen Menge zugesetzt. Das Verhältnis von Platinmetall, z.B. Palladium, zu Base wird vorzugsweise so gewählt, daß pro Grammatom Platinmetall, z.B. Palladium, 0,1 bis 500, bevorzugt 0,3 bis 200 besonders bevorzugt 0,9 bis 130 Äquivalente Base eingesetzt werden.

Das Verfahren wird vorzugsweise ohne Lösungsmittel durchgeführt. Selbstverständlich können auch inerte Lösungsmittel verwendet werden. Als Beispiele für Lösungsmittel seien Dimethylacetamid, N-Methylpyrrolidinon, t-Butanol, Cumylalkohol, Isoamylalkohol, Tetramethylhamstoff, Diethylenglykol, halogenierte Kohlenwasserstoffe (z.B. Chlorbenzol oder Dichlorbenzol) und Ether, wie Dioxan, Tetrahydrofuran, t-Butylmethylether und veretherte Glykole, genannt.

Geeignete Platinmetall-Katalysatoren bestehen aus mindestens einem Edelmetall der Gruppe VIII, vorzugsweise Palladium. Es kann in verschiedener Form zugegeben werden. Palladium kann in metallischer Form oder bevorzugt in Form von PalladiumVerbindungen der Oxidationsstufen 0 und +2, wie beispielsweise Palladium(II)-acetylacetonat, -halogenide, -carboxylate von C₂-C₆-Carbonsäuren, -nitrat, -oxide oder Palladiumkomplexe, die beispielsweise Olefine, Amine, Phosphine und Halogenide enthalten können, eingesetzt werden. Besonders bevorzugt sind Palladiumbromid und Palladiumacetylacetonat.

Die Menge an Platinmetall-Katalysator ist nicht beschränkt. Meist wird so viel Katalysator zugesetzt, daß die Konzentration des Metalls im Reaktionsansatz 1 - 3000 ppm beträgt, bevorzugt sind Konzentrationen von 5 - 500 ppm.

Als Cokatalysator wird ein Metall der Gruppen III A, III B, IV A, IV B, V B, I B, II B, VI B, VII B, der Seltenerdmetalle (Atomnummern 58-71) oder der Eisengruppe des Periodensystems der Elemente (Mendelejew) eingesetzt, wobei das Metall in verschiedenen Oxidationsstufen eingesetzt werden kann. Bevorzugt werden Mn, Cu, Co, V, Zn, Ce und Mo eingesetzt, z.B. Mangan(II), Mangan(III), Kupfer(I), Kupfer(II), Kobalt(II), Kobalt(III), Vanadium(III) und Vanadium(IV). Die Metalle können beispielsweise als Halogenide, Oxide, Carboxylate von C₂-C₆-Carbonsäuren, Diketonate oder Nitrate sowie als Komplexverbindungen, die beispielsweise Kohlenmonoxid, Olefine, Amine, Phosphine und Halogenide enthalten können, eingesetzt werden. Besonders bevorzugt werden Mn, Cu, Mo und Ce eingesetzt. Ganz besonders bevorzugt werden Manganverbindungen im erfindungsgemäßen Verfahren verwendet, besonders bevorzugt Mangan(II)komplexe, ganz besonders bevorzugt Mangan(II)acetylacetonat bzw. Mangan(III)-acetylacetonat.

Der Cokatalysator wird in einer solchen Menge zugesetzt, daß seine Konzentration im Bereich von 0,0001 bis 20 Gew.-% des Reaktionsgemisches liegt, bevorzugt ist der Konzentrationsbereich von 0,005 bis 5 Gew.-%, besonders bevorzugt 0,01 bis 2 Gew.-%.

Bei den quatemären Salzen kann es sich beispielsweise um mit organischen Resten substituierte Ammonium-, Guanidinium-, Phosphonium- oder Sulfoniumsalze handeln. Geeignet sind Ammonium-, Guanidinium-, Phosphonium- und Sulfoniumsalze, die als organische Reste C₆- bis C₁₀-Aryl-, C₇- bis C₁₂-Aralkyl- und/oder C₁- bis C₂₀-Alkyl-Reste und als Anion ein Halogenid, Tetrafluoroborat oder Hexafluorophosphat tragen. Bevorzugt werden Ammoniumsalze eingesetzt, die als organische Reste C₆- bis C₁₀-Aryl-, C₇- bis C₁₂-Aralkyl- und/oder C₁- bis C₂₀-Alkyl-Reste und als Anion ein Halogenid tragen, besonders bevorzugt ist Tetrabutylammoniumbromid. Die Menge eines solchen quatemären Salzes kann beispielsweise 0,1 - 20 Gew.-%, bezogen auf das Gewicht des Reaktionsgemisches, betragen. Vorzugsweise beträgt diese Menge 0,5 - 15 Gew.-%, besonders bevorzugt 1 - 5 Gew.-%.

Für die Diarylcarbonatherstellung können homogene Katalysatorsysteme eingesetzt werden oder heterogene Katalysatoren, bei denen das Platinmetall oder das Platinmetall und der Cokatalysator auf einem heterogenen Träger aufgebracht sind. Bei den heterogenen Katalysatorsystemen sind die übrigen Komponenten des Katalysatorsystems, wie die Base, die quaternäre Verbindung und gegebenenfalls der Cokatalysator, weiterhin in der Reaktionslösung homogen gelöst.

Der heterogene Trägerkatalysator kann ortsfest in Rührbehältern, Blasensäulen, einem Rieselphasenreaktor oder Kaskaden dieser Reaktoren eingesetzt werden. Eine Abtrennung des Trägerkatalysators aus dem Reaktionsgemisch entfällt dann völlig.

Bei der Herstellung von Diarylcarbonaten durch Direktcarbonylierung von aromatischen Hydroxyverbindungen wird eine Diarylcarbonat, aromatische Hydroxyverbindung, Wasser, Base und quaternäres Salz enthaltende Reaktionsmischung erhalten. Wird ein homogenes Katalysatorsystem verwendet, so enthält die Reaktionsmischung außerdem noch Platinmetallkatalysator und Cokatalysator.

Die Reaktionsmischung wird nach der Reaktion bei Drücken von 1 bis 100 mbar, bevorzugt 5 bis 50 mbar, besonders bevorzugt 10 bis 40 mbar, und Temperaturen von 80 bis 160°C, bevorzugt 100 bis 140°C aufgetrennt in eine Diarylcarbonat, aromatische Hydroxyverbindung, Base und quatemäres Salz sowie gegebenenfalls weitere Katalysatorbestandteile enthaltende Flüssigphase und eine Diarylcarbonat, aromatische Hydroxyverbindung und Wasser enthaltende Gasphase. Wichtig ist dabei, daß die Auftrennung möglichst schnell durchgeführt wird, so daß die Reaktionsmischung nur kurze Zeit höheren Temperaturen ausgesetzt ist. Daher wird die Auftrennung in einer Destillationsapparatur ausgeführt, die nur eine theoretische Trennstufe aufweist. So gelingt es, eine Gasphase zu erhalten, die neben aromatischer Hydroxyverbindung und Wasser auch Diarylcarbonat enthält. Die Auftrennung der Reaktionsmischung erfolgt bevorzugt in einem Fallfilmverdampfer, Dünnschichtverdampfer oder einem Verdampfer mit Zwangsumlauf und innen oder außen liegenden Heizregistern. Dabei werden Flüssig- und Gasphase bevorzugt im Gleichstrom geführt. Bei der Auftrennung der Reaktionsmischung wird das in der Reaktionsmischung enthaltene Wasser möglichst vollständig in die Gasphase überführt. Außerdem wird ein Teil des in der Reaktionsmischung enthaltenen Diarylcarbonats schonend in die Gasphase überführt, aus der es anschließend in reiner Form isoliert werden kann.

Bei der Auftrennung werden bevorzugt etwa 50 bis 90 Gew.-% der ursprünglichen Reaktionsmischung in die Gasphase überführt. Der Anteil der erhaltenen Flüssigphase beträgt etwa 10 bis 50 Gew.-% der eingesetzten Menge Reaktionsmischung. Wird der Anteil der Flüssigphase kleiner als 10 Gew.-%, so besteht die Gefahr, daß vermehrt Nebenreaktionen auftreten können, die zur Desaktivierung des Katalysators und zu geringerer Ausbeute an Diarylcarbonat führen.

Die Flüssigphase kann dem Reaktionsschritt der Direktcarbonylierung ohne weitere Aufarbeitung wieder zugeführt werden. Bei der Verwendung homogener Katalysatorsysteme kann es vorteilhaft sein, vor der Rückführung der Flüssigphase desaktivierte Katalysatoranteile zu entfernen, beispielsweise durch Filtration.

Die bei der Auftrennung der Reaktionsmischung erhaltene Gasphase wird anschließend weiter aufgearbeitet. Bevorzugt geschieht dies durch fraktionierende Kondensation. Hierbei wird eine im wesentlichen aus Diarylcarbonat und aromatischer Hydroxyverbindung bestehende Flüssigphase und eine im wesentlichen aus aromatischer Hydroxyverbindung und Wasser bestehende Gasphase erhalten. Die fraktionierende Kondensation wird vorzugsweise bei Temperaturen von 40 bis 120°C, besonders bevorzugt 60 bis 100°C durchgeführt.

Bevorzugt wird die hierbei erhaltene Flüssigphase anschließend destillativ von der aromatischen Hydroxyverbindung befreit. Das erhaltene Diarylcarbonat kann anschließend einer weiteren Reinigung unterzogen werden, z.B. gewaschen, mit Adsorbentien behandelt, destilliert oder kristallisiert werden. Bevorzugt wird die abgetrennte aromatische Hydroxyverbindung dem Reaktionsschritt der Direktcarbonylierung wieder zugeführt.

Bevorzugt wird man aus der bei der Ruftrennung der Reaktionsmischung erhaltenen, im wesentlichen aus aromatischer Hydroxyverbindung und Wasser bestehenden Gasphase die aromatische Hydroxyverbindung isolieren und dem Reaktionsschritt der Direktcarbonylierung wieder zuführen. Die Isolierung der aromatischen Hydroxyverbindung aus der Gasphase erfolgt bevorzugt durch vollständige Kondensation und anschließende destillative Abtrennung leichtsiedender Verbindungen, insbesondere des Wassers, von der aromatischen Hydroxyverbindung.

### Beispiele

### Beispiel 1

Es wurde eine Apparatur verwendet, die aufgebaut war aus einem Autoklaven mit kontinuierlicher Gas- und Flüssigkeitsdosierung, Druckhalteeinrichtung sowie kontinuierlichem Flüssigkeitsaustrag, der über ein Puffergefäß direkt mit einem angeschlossenen Dünnschichtverdampfer und der Dünnschichtverdampfersumpf über eine Rückführleitung mit der Flüssigkeitsdosierung verbunden war.

Das kontinuierlich abgeführte Abgas des Autoklaven gelangte nach der Entspannung über nachgeschaltete Kühlfallen in online-Analysatoren für CO₂ und O₂. Die Flüssigkeitsdosierung bestand aus drei kontinuierlichen Dosiereinheiten. Die nach dem Dünnschichtverdampfer erhaltenen Brüden wurden kondensiert.

In den Autoklaven wurden bei ca. 85°C und 14 bar über zwei Dosierpumpen pro Stunde 1200g einer Reaktionslösung bestehend aus ca. 120 ppm Pd (als PdBr₂), ca. 300 ppm Mn (als Mn(acac)₂), ca. 2,5 Gew.-% TBAB (Tetrabutylammoniumbromid), ca. 1,5 Gew.-% TBAP (Tetrabutylammoniumphenolat) und Phenol eindosiert. Der Flüssigkeitsstand im Reaktor wurde auf ca. 1200 ml eingestellt. Gleichzeitig wurde in den Autoklaven ca. 700 NL/h eines Gasgemisches bestehend aus 2.5 Vol.% Sauerstoff, 1,5 Vol.% Inertgas (N₂, Ar usw.) und Rest zu 100% Kohlenmonoxid eindosiert. Das Abgas wurde in den Kühlfallen vom Reaktionswasser und Phenol befreit und den Analysatoren zugeführt.

Der Flüssigkeitsstrom aus dem Reaktor wurde kontinuierlich in den bei ca. 20 mbar und 120°C betriebenen Dünnschichtverdampfer überführt. Das den Dünnschichtverdampfer verlassende Sumpfprodukt wurde gesammelt, die Brüden bei ca. 42°C mittels Dephlegmatoren kondensiert. Nachdem genügend Sumpfprodukt gesammelt worden war, wurde die Lösung nach Filtration und Verdünnung mit Frischphenol über die dritte Dosierpumpe dem Reaktor wieder zugeführt. Gleichzeitig wurden die Fördermengen der ersten beiden Dosierpumpen und die Konzentrationen der Katalysatorkomponenten so eingestellt, daß die Konzentrationen und Mengenströme des Reaktorzulaufs den oben angegebenen Werten entsprach. Die Katalysatorergänzungen entsprachen den abfiltrierten bzw. abdestillierten Mengenströmen. Im weiteren Verlauf wurde das Sumpfprodukt des Dünnschichtverdampfers ständig wie oben beschrieben der Reaktion wieder zugeführt. Die entnommenen DPC-, PhOHund Wasser-Mengen wurden dem System in Form von Frischphenol, aufgeteilt auf die drei Dosierungen, wieder zugeführt Durch schrittweise Erhöhung der Temperatur des Dünnschichtverdampfers auf ca. 140°C bei gleichem Vakuum wurde ein DPC-Gehalt im Zulaufstrom des Reaktors von ca. 9 - 10 Gew.-% DPC eingestellt. Nach ca. 64 h war die Apparatur im Gleichgewicht, die DPC-Konzentration im Zulauf des Dünnschichtverdampfers betrug ca. 19 Gew.-%. In den Kühlfallen kondensierte stündlich etwa 6,3 g eines Phenol/Wasser-Gemischs. Mit dem Dünnschichtverdampfer wurde die pro Stunde im Reaktor gebildete DPC-Menge, gemeinsam mit Phenol und restlichem Reaktionswasser abdestilliert. Das zwischen Destillat und Sumpf Verhältnis im Dünnschichtverdampfer betrug ca. 5,5 : 1. Der unverdünnte Sumpf des Dünnschichtverdampfers (vor Filtration, Verdünnung mit Frischphenol und Rückführung) bestand zu ca. 10 Gew.-% aus Phenol, zu ca. 56 Gew.-% aus DPC und enthielt ca. 25 Gew.-% Katalysatorsystem. Der restliche Teil des Sumpfes bestand aus schwersiedenden Nebenprodukten.

Die Analyse der Reaktionsprodukte erfolgte mittels HPLC. Die Raum-Zeit-Ausbeute an DPC, bezogen auf den Reaktorinhalt, betrug 96 g DPC/lh. Verkrustungen oder DPC-Verluste wurden nicht beobachtet.

### Vergleichsbeispiel 2

Aus einem Versuchslauf analog Beispiel 1 wurden vor dem Verdampfer 1,2 1 Reaktionslösung aus der im Gleichgewicht befindlichen Apparatur entnommen. Der DPC-Gehalt der Lösung betrug 19,3 Gew.-% (HPLC-Analyse). Die destillative Aufarbeitung der Reaktionslösung erfolgte wie in Beispiel 1 der EP-B 507 546 beschrieben.

Bei ca. 15 Torr und 100°C Kolbeninnentemperatur destillierte zunächst Phenol ab. Als im Vorlagekolben kein Destillat mehr kondensierte, wurde unter Stickstoffbeschleierung der Vorlagekolben ausgetauscht. Die Apparatur wurde erneut auf ein Vakuum von 15 Torr eingestellt und die Temperatur auf 165°C erhöht. Da kaum Destillat auftrat, wurde die Temperatur im Kolben in 5°C Schritten erhöht. Während der Destillation mußte die Temperatur des Kolbeninhalts ständig erhöht werden, bis bei 205°C kein Destillat mehr auftrat.

Die Massenbilanz und Analyse der einzelnen Fraktionen und des Destillationsrückstands ergab, daß fast das gesamte Tetrabutylammoniumbromid und -phenolat bei der Destillation zerstört worden war. Der Destillationssumpf ( 4 % der Eduktmenge) bestand im wesentlichen aus DPC, Hochsiedern und Katalysatorbestandteilen. Das abgetrennte Phenol der ersten Fraktion besaß eine GC-Reinheit von 99,7 %. Die zweite Fraktion bestand lediglich zu ca. 40% aus DPC, der Rest waren Phenol, Tributylamin und Schwersieder. Die Massenbilanz ergab deutlich mehr Phenol und Nebenprodukte und nur 68 % der im Edukt vorhandenen DPC-Menge.

Aufgrund dieser Ergebnisse ist eine wirtschaftliche Anwendung dieser in EP-B 507 546 beschriebenen Aufarbeitungsmethode nicht möglich, da neben dem DPC-Verlust das Katalysatorsystem nicht oder nur mit erheblichen Aufwand und unter vollständigem Verlust des eingesetzten TBAB und TBAP rückführbar ist.

### Beispiel 3

Es wurde die Apparatur wie in Beispiel 1 beschrieben benutzt, der Dünnschichtverdampfer wurde aber durch einen Fallfilmverdampfer ersetzt und es wurde zusätzlich eine Aufarbeitung der Brüden durchgeführt.

Die Aufarbeitung der Fallfilmverdampfer-Brüden erfolgte durch fraktionierende Kondensation in einem Dephlegmator bei ca. 80°C, wobei sich das Teilkondensat in einem Glaskolben mit Bodenablaßventil und Vakuumschleuse kontinuierlich sammelte. Die unkondensierten Brüden, die im wesentlichen aus Phenol und restlichem Reaktionswasser bestanden, gelangten anschließend zur Totalkondensation (ca. 44°C), bestehend aus zwei auf einem Glaskolben mit Bodenablaßventil und Vakuumschleuse. Restliches Phenol, Wasser und Leichtsieder wurden mit Hilfe einer Kühlfallenstrecke vor der Vakuumpumpe ausgefroren.

Die Kühlfalleninhalte und der Inhalt des Kolbens unter der Totalkondensation wurden kontinuierlich in einer Vakuumdestillationskolonne (verspiegelte Füllkörperkolonne, ca. 2 m Höhe und 50 mm Durchmesser, mit mittiger Edukteinspeisung, aufgesetztem Dampfteiler zur Destillatabnahme und Naturumlaufverdampfer als Sumpfheizung) eingespeist und bei ca. 170°C Sumpftemperatur und 200 mbar destilliert.

Das DPC/Phenol-Gemisch unter dem Kondensator wurde in einer zweiten Destillationskolonne, wie obige Kolonne aber mit einem Durchmesser von 25 mm, bei 200°C Sumpftemperatur und 10 mbar destilliert.

Die Apparatur wurde wie in Beispiel 1 beschrieben angefahren, wobei 0,85 Gew.-% Tributylamin statt 1,5 Gew.-% Tetrabutylammoniumphenolat als Base benutzt wurden.

Als eine ausreichende Menge an Produkten für die Destillationskolonnen vorhanden war, wurden diese nacheinander angefahren und kontinuierlich betrieben. Nach ca. 207 h war die Apparatur im Gleichgewicht. In den Kühlfallen kondensierte stündlich etwa 7,1 g eines Phenol/Wasser-Gemischs. Mit dem Fallfilmverdampfer wurde, wie in Beispiel 1 mit dem Dünnschichtverdampfer, die pro Stunde im Reaktor gebildete DPC-Menge, gemeinsam mit Phenol und restlichem Reaktionswasser abdestilliert.

Das Verhältnis der Fallfilmer-Destillate zum Fallfilmer-Sumpf betrug ca. 5,4 : 1. Der unverdünnte Fallfilmersumpf (vor Filtration, Verdünnung mit Frischphenol und Rückführung) bestand zu ca. 9,8 Gew.-% aus Phenol, zu ca. 56 Gew.-% aus DPC, enthielt ca. 20 Gew.-% Katalysatorsystem. Der restliche Teil des Sumpfes bestand aus schwersiedenden Nebenprodukten.

Das Verhältnis von Teilkondensat zu Totalkondensat (mit Kühlfalleninhalt) betrug 1 : 3,13. Das Teilkondensat bestand aus einem wasserfreien Gemisch aus ca. 45 Gew.-% DPC und ca. 55 Gew.-% Phenol sowie Spuren weiterer Stoffe. Als Sumpfprodukt bei der destillativen PhOH/DPC-Trennung wurde ein Roh-DPC mit > 99,5 Gew.-% DPC erhalten. Das Kopfprodukt bestand zu > 99 Gew.-% aus PhOH, etwas TBA und Spuren weiterer Verunreinigungen.

Das Totalkondensat bestand aus Phenol, etwas Tributylamin, restlichem Reaktionswasser, etwas DPC und Spuren leichtsiedener Nebenprodukte. Die destillative Aufarbeitung des Totalkondensats ergab als Sumpfprodukt ein wasserfreies ca. 98,8 Gew.-%iges Phenol, was im wesentlichen mit DPC und Tributylamin verunreinigt war. Als Destillat kondensierte Wasser mit geringen Mengen Phenol und Spuren von Leichtsiedern. Das Verhältnis zwischen Sumpf und Destillat betrug etwa 47,5 : 1.

Die beiden abdestillierten Phenolströme der Destillationskolonnen wurden vereinigt und zur Verdünnung des Sumpfprodukts des Fallfilmverdampfers verwendet; ein Teilstrom wurde gemeinsam mit Frischphenol für die Katalysatorergänzungen in Dosierung 1 und 2 eingesetzt.

Die Analyse der Reaktionsprodukte erfolgte mittels HPLC. Die Raum-Zeit-Ausbeute an DPC, bezogen auf den Reaktorinhalt, betrug ca. 90 g DPC/lh. Verkrustungen oder DPC-Verluste wurden nicht beobachtet.

## Patentansprüche

1. Verfahren zur Aufarbeitung von Reaktionsmischungen aus der Herstellung von Diarylcarbonaten durch Direktcarbonylierung von aromatischen Hydroxyverbindungen, bei dem eine Diarylcarbonat, aromatische Hydroxyverbindung, Wasser, Base und quaternäres Salz enthaltende Reaktionsmischung in einer nur eine theoretische Trennstufe aufweisenden Destillationsapparatur bei Drücken von 1 bis 100 mbar und Temperaturen von 80 bis 160°C aufgetrennt wird in eine Diarylcarbonat, aromatische Hydroxyverbindung, Base und quaternäres Salz enthaltende Flüssigphase und eine Diarylcarbonat, aromatische Hydroxyverbindung und Wasser enthaltende Gasphase, die Flüssigphase dem Reaktionsschritt der Direktcarbonylierung ohne weitere Aufarbeitung wieder zugeführt und die Gasphase anschließend weiter aufgearbeitet wird.

2. Verfahren gemäß Anspruch 1, bei dem die Reaktionsmischung einen Platinmetallkatalysator und einen Cokatalysator enthält, die nach der Auftrennung in der Flüssigphase verbleiben und mit dieser, gegebenenfalls nach Abtrennung von desaktivierten Katalysatorbestandteilen, dem Reaktionsschritt der Direktcarbonylierung ohne weitere Aufarbeitung wieder zugeführt werden.

3. Verfahren gemäß Anspruch 1 oder 2, bei dem die Ruftrennung der Reaktionsmischung in einem Fallfilmverdampfer, Dünnschichtverdampfer oder einem Verdampfer mit Zwangsumlauf und innen oder außen liegenden Heizregistern erfolgt.

4. Verfahren gemäß einem der Ansprüche 1 bis 3, bei dem die erhaltene Gasphase durch fraktionierte Kondensation in eine im wesentlichen aus Diarylcarbonat und aromatischer Hydroxyverbindung bestehende Flüssigphase und eine im wesentlichen aus aromatischer Hydroxyverbindung und Wasser bestehende Gasphase aufgetrennt wird.

5. Verfahren gemäß Anspruch 4, bei dem die erhaltene Flüssigphase destillativ von der aromatischen Hydroxyverbindung befreit und das erhaltene Diarylcarbonat gegebenenfalls einer weiteren Reinigung unterzogen wird.

6. Verfahren gemäß Anspruch 5, bei dem die abgetrennte aromatische Hydroxyverbindung dem Reaktionsschritt der Direktcarbonylierung wieder zugeführt wird.

7. Verfahren gemäß einem der Ansprüche 4 bis 6, bei dem aus der im wesentlichen aus aromatischer Hydroxyverbindung und Wasser bestehenden Gasphase die aromatische Hydroxyverbindung isoliert und dem Reaktionsschritt der Direktcarbonylierung wieder zugeführt wird.

## Claims

1. A process for the work up of reaction mixtures from the preparation of diaryl carbonates by direct carbonylation of aromatic hydroxy compounds, wherein a reaction mixture containing diaryl carbonate, aromatic hydroxy compound, water, base and quaternary salt is separated in a distillation apparatus having only one theoretical separation stage at pressures from 1 to 100 mbar and at temperatures from 80 to 160 °C into a liquid phase containing diaryl carbonate, aromatic hydroxy compound, base and quaternary salt and a gas phase containing diaryl carbonate, aromatic hydroxy compound and water, the liquid phase is recycled without further work up to the reaction step of direct carbonylation and the gas phase then undergoes further work up.

2. A process according to claim 1, wherein the reaction mixture contains a platinum metal catalyst and a cocatalyst which remain in the liquid phase after separation and are recycled with said liquid phase, optionally after separation of deactivated catalyst constituents, to the reaction step of direct carbonylation without further work up.

3. A process according to claim 1 or 2, wherein the separation of the reaction mixture takes place in a falling-film evaporator, thin-film evaporator or a forced circulation evaporator with internal or external heating elements.

4. A process according to one of claims 1 to 3, wherein the gas phase obtained is separated by fractional condensation into a liquid phase composed substantially of diaryl carbonate and aromatic hydroxy compound and a gas phase composed substantially of aromatic hydroxy compound and water.

5. A process according to claim 4, wherein the aromatic hydroxy compound is removed by distillation from the liquid phase obtained and the diaryl carbonate obtained optionally undergoes a further purification.

6. A process according to claim 5, wherein the separated aromatic hydroxy compound is recycled to the reaction step of direct carbonylation.

7. A process according to one of claims 4 to 6, wherein the aromatic hydroxy compound is isolated from the gas phase composed substantially of aromatic hydroxy compound and water and recycled to the reaction step of direct carbonylation.

## Revendications

1. Procédé de traitement de mélanges réactionnels provenant de la préparation de carbonates de diaryle par carbonylation directe de composés hydroxylés aromatiques, dans lequel un mélange réactionnel contenant le carbonate de diaryle, le composé hydroxylé aromatique, de l'eau, une base et un sel quaternaire est partagé dans un appareil de distillation ne présentant qu'un plateau théorique de séparation à des pressions allant de 1 à 100 mbar et des températures allant de 80 à 160°C, en une phase liquide contenant le carbonate de diaryle, le composé hydroxylé aromatique, la base et le sel quaternaire et une phase gazeuse contenant le carbonate de diaryle, le composé hydroxylé aromatique et l'eau, la phase liquide est ramenée à l'étape de réaction de la carbonylation directe sans autre traitement et la phase gazeuse est ensuite encore traitée.

2. Procédé suivant la revendication 1, dans lequel le mélange réactionnel contient un catalyseur métal de type platine et un cocatalyseur, qui restent dans la phase liquide après la séparation et sont ramenés avec celle-ci, le cas échéant après séparation des constituants désactivés du catalyseur, à l'étape de réaction de la carbonylation directe sans autre traitement.

3. Procédé suivant la revendication 1 ou 2, dans lequel le partage du mélange de réaction est réalisé dans un évaporateur à film tombant, un évaporateur sur couche mince ou un évaporateur avec circulation forcée et registre de tirage interne ou externe.

4. Procédé suivant l'une quelconque des revendications 1 à 3, dans lequel la phase gazeuse obtenue est partagée par condensation fractionnée en une phase liquide consistant essentiellement en carbonate de diaryle et composé hydroxylé aromatique et une phase gazeuse consistant essentiellement en composé hydroxylé aromatique et eau.

5. Procédé suivant la revendication 4, dans lequel la phase liquide obtenue est libérée par distillation du composé hydroxylé aromatique et le carbonate de diaryle obtenu est soumis le cas échéant, à une autre purification.

6. Procédé suivant la revendication 5, dans lequel le composé hydroxylé aromatique séparé est ramené dans l'étape de réaction de la carbonylation directe.

7. Procédé suivant l'une quelconque des revendications 4 à 6, dans lequel le composé hydroxylé aromatique est isolé de la phase gazeuse consistant essentiellement en composé hydroxylé aromatique et eau et est ramené dans l'étape de réaction de la carbonylation directe.
